Europäisches Patentamt

(19) European Patent Office

Office européen des brevets

(11) Numéro de publication : **0 288 336 B1**

(12) **FASCICULE DE BREVET EUROPEEN**

(45) Date de publication du fascicule du brevet :
**27.11.91 Bulletin 91/48**

(21) Numéro de dépôt : **88400625.5**

(22) Date de dépôt : **16.03.88**

(51) Int. Cl.⁵ : $A61K\ 9/70$, $A61L\ 15/20$, $A61K\ 47/00$

(54) **Dispositif auto-adhésif d'administration d'un principe actif par voie percutanée.**

(30) Priorité : **25.03.87 FR 8704133**

(43) Date de publication de la demande :
**26.10.88 Bulletin 88/43**

(45) Mention de la délivrance du brevet :
**27.11.91 Bulletin 91/48**

(84) Etats contractants désignés :
**AT BE CH DE ES FR GB GR IT LI LU NL SE**

(56) Documents cités :
**EP-A- 0 159 168
PATENT ABSTRACTS OF JAPAN, vol. 10, no. 144 (C-349)[2201], 27 May 1986**

(73) Titulaire : **LABORATOIRES D'HYGIENE ET DE DIETETIQUE L.H.D. Société Anonyme dite:
38 avenue Hoche
F-75008 Paris (FR)**

(72) Inventeur : **Guillemet, Alain
12, rue Martin de Noinville
F-21000 Dijon (FR)**
Inventeur : **Teillaud, Eric
1, allée Roger Bernard
F-21240 Talant (FR)**
Inventeur : **Reginault, Philippe
13, rue de Provence
F-21121 Fontaine-les-Dijon (FR)**

(74) Mandataire : **Clisci, Serge et al
S.A. FEDIT-LORIOT CONSEILS EN PROPRIETE INDUSTRIELLE 38, avenue Hoche
F-75008 Paris (FR)**

EP 0 288 336 B1

## Description

La présente invention concerne une nouvelle matrice pour l'administration d'un principe actif par voie percutanée.

Par matrice, on entend ici la composition chimique des ingrédients permettant l'administration percutanée d'un principe actif, et par dispositif, on entend l'ensemble constitué par la matrice et son support.

Il a déjà été proposé de nombreux systèmes pour l'administration d'un principe actif par voie percutanée. Japan, 10 No 144 (C-349) [2201], relatif au document JP-A-615012, une composition adhésive à la peau d'ingrédients non solubles dans l'eau contenant un copolymère éthylène/acétate de vinyle. On connaît en particulier des systèmes destinés à administrer de la trinitroglycérine par voie percutanée pour le traitement de l'angine de poitrine. Ces systèmes, qui sont constitués d'un support sur lequel est déposé un réservoir ou une matrice contenant le principe actif, présentent l'inconvénient d'une adhésivité à la peau qui décroît rapidement au cours du temps. En effet, dans le cas d'un réservoir, le principe actif est dissous dans un solvant servant de vecteur de transport du principe actif à travers une membrane microporeuse vers la peau. Dans le cas d'une matrice, le principe actif contenu dans un réseau polymérique est également dissous dans un solvant servant de vecteur de transport. Le réservoir ou la matrice étant maintenu sur la peau par un adhésif classique, du type masse acrylique, le solvant dissout partiellement certains composants de l'adhésif qui perd ainsi rapidement son efficacité.

Pour remédier à cet inconvénient, il a déjà été proposé, dans EP-A-0159168, une solution telle que la matrice contenant le principe actif a des propriétés d'auto-adhérence à la peau. Cette matrice, contenant un principe actif, est constituée d'une protéine soluble dans l'eau, d'un alcool polyhydrique, d'un agent tackifiant et d'une substance oléagineuse. Plus particulièrement la protéine soluble dans l'eau peut être naturelle ou synthétique, animale ou végétale, comme par exemple la gélatine, le collagène, la caséine ou la glu, dans des proportions de 5 à 50% ; l'alcool polyhydrique peut être un glycol, comme par exemple l'éthylène glycol, le propylène glycol, le butylène glycol ou le polyéthylène glycol, un triol ou un polyol, dans des proportions de 5 à 50% ; l'agent tackifiant peut être de la cellulose ou un dérivé, comme par exemple la méthylcellulose, l'éthylcellulose, la propylcellulose, la méthylpropylcellulose, l'hydroxyméthylcellulose, l'hydroxyéthylcellulose, l'hydroxy-propylcellulose ou la carboxyméthylcellulose, un polysaccharide, l'alcool polyvinylique ou la polyvinylpyrrolidone, dans des proportions de 0,1 à 15% ; et la substance oléagineuse peut être un ester d'acide gras, la paraffine, la lanoline, un alcool aliphatique supérieur, comme par exemple les alcools octyldodécylique, palmitique, stéarique ou myristique, ou l'huile de silicone, dans des proportions de 0,1 à 25%.

On connaît par ailleurs du résumé Pat. Abs. of Japan, 10 No 144 (C-349) [2201], relatif au document JP-A-615012, une composition adhésive à la peau d'ingrédients non solubles dans l'eau contenant un copolymère éthylène/acétate de vinyle.

On propose à présent une nouvelle solution technique faisant appel à une matrice auto-adhésive différente de l'art antérieur rappelé ci-dessus, et notamment de EP-A-0159168, par la nature du constituant principal, par les proportions des autres constituants, ainsi que par la facilité de mise en oeuvre industrielle.

La matrice auto-adhésive pour l'administration d'un principe actif par voie percutanée selon l'invention est caractérisée en ce qu'elle comprend :

a) 40 à 60 parties en poids de copolymère d'éthylène et d'acétate de vinyle,

b) 40 à 60 parties en poids d'alcool aliphatique supérieur,

c) 1 à 20 parties en poids de dérivé de cellulose,

d) 0,1 à 8 parties en poids d'alcool polyhydrique, et

e) 0,01 à 10 parties en poids de principe actif administrable par voie percutanée,

le rapport en poids a + c/b + d étant compris entre 0,7 et 1,3.

De façon avantageuse, on utilisera un copolymère d'éthylène et d'acétate de vinyle (EVA) ayant une teneur en motifs acétate de vinyle comprise entre 35 et 55% en poids, de préférence de l'ordre de 45% en poids, par rapport au poids du copolymère d'éthylène/acétate de vinyle.

Par alcool aliphatique supérieur, on entend ici les mono-alcools saturés ou insaturés ayant 12 à 20 atomes de carbone comme par exemple le 2-octyl-1-dodécanol, l'alcool palmitique, l'alcool stéarique ou l'alcool myristique.

Par dérivé de cellulose, on entend ici les alkylcelluloses, comme par exemple le méthylcellulose, l'éthylcellulose, la propylcellulose ou la méthylpropylcellulose et les hydroxyalkylcelluloses, comme par exemple l'hydroxyméthylcellulose, l'hydroxyéthylcellulose ou l'hydroxypropylcellulose.

Par alcool polyhydrique, on entend ici les glycols, comme par exemple l'éthylène glycol, le propylène glycol, le butylène glycol, le triéthylène glycol, le diéthylène glycol ou le polyéthylène glycol ou le polypropylène glycol.

Par principe actif, on entend ici tout produit percutanéable solide ou liquide qui est soluble, au moins partiellement, dans la phase alcool polyhydrique-alcool aliphatique supérieur. On préférera les stéroïdes et plus particulièrement l'oestradiol, notamment le β-oestradiol, les dérivés de l'oestradiol, notamment les acétates comme par exemple le 17-acétate oestradiol ou le 3,17-diacétate oestradiol, la progestérone et ses dérivés, la testostérone et ses dérivés et les corticostéroïdes.

Pratiquement, on préférera une teneur en principe actif telle que tout ledit principe actif soit dissous dans la phase alcool polyhydrique — alcool aliphatique supérieur et on préférera les formulations telles que le rapport de la phase solvant (alcool aliphatique supérieur — alcool polyhydrique) à la phase polymère (EVA — dérivé de cellulose) soit voisin de l'unité.

Selon un mode préféré de réalisation de l'invention, le dispositif auto-adhésif d'administration d'un principe actif par voie percutanée est caractérisé en ce qu'il comprend un support enduit d'une matrice constituée de :

    a) 40 à 60 parties en poids de copolymère d'éthylène et d'acétate de vinyle,

    b) 40 à 60 parties en poids de 2-octyl-1-dodécanol,

    c) 1 à 20 parties en poids d'éthylcellulose,

    d) 0,1 à 8 parties en poids de dipropylène glycol, et,

    e) 0,01 à 10 parties en poids d'oestradiol,

    le rapport en poids a + c/b + d étant compris entre 0,7 et 1,3.

Plus précisément selon ce mode préféré de réalisation de l'invention, on utilisera de façon avantageuse 45 parties en poids d'EVA ayant une teneur en motifs acétate de vinyle de l'ordre de 45%, 40 à 45 parties en poids de 2-octyl-1-dodécanol, 5 à 10 parties en poids d'éthylcellulose de viscosité comprise entre $2 \times 10^{-2}$ et $2 \times 10^{-1}$ Pa · s, 1 à 5 parties en poids de dipropylène glycol et 3 à 5 parties en poids de β-oestradiol.

Le support recevant la matrice pourra être tout support souple, imperméable aux constituants de la matrice et d'épaisseur comprise entre 50 et 500 micromètres. On préférera un support polymérique tel que par exemple le polyéthylène, le polypropylène, les polyesters, les mousses, notamment microcellulaires, de polyéthylène connues notamment dans le domaine des pansements.

De façon pratique, la matrice pourra être recouverte d'une pellicule de protection, pelable avant utilisation du dispositif, lequel pourra être lui-même emballé dans une protection étanche comme par exemple les complexes polyéthylène-aluminium.

Le dispositif auto-adhésif d'administration d'un principe actif par voie percutanée selon l'invention, non collant immédiatement au contact de la peau, est doué d'un pouvoir adhésif progressif se renforçant dès quelques minutes après mise en place sur la peau d'un sujet. Le dispositif ou la matrice peut être facilement enlevé sans desquamation de la peau puis reposé en gardant un pouvoir adhésif identique. Ceci est particulièrement avantageux pour un tel dispositif devant être gardé plusieurs jours par un sujet qui peut ainsi enlever momentanément ledit dispositif, par exemple lors de la prise d'un bain.

Le dispositif selon l'invention présente encore l'avantage d'être réduit à une surface et une épaisseur minimales. De par son propre pouvoir adhésif, il n'est pas nécessaire de border la partie active du dispositif par une masse adhésive. Toute la surface du dispositif est à la fois active et adhésive. Des épaisseurs de quelques centaines de micromètres de matrice (150 à 250 micromètres) suffiront pour délivrer à travers la peau pendant plusieurs jours la quantité nécessaire de principe actif.

Suivant l'invention, on préconise un procédé pour la préparation d'une matrice auto-adhésive pour administration percutanée qui comprend les étapes suivantes :

    1) on mélange sous agitation le moyen a) et une portion du moyen b) inférieure à la quantité requise de 40 à 60 parties en poids, à une température supérieure ou égale à 110°C,

    2) on incorpore, sous agitation, à une température supérieure ou égale à 110°C, le moyen c) dans le mélange résultant du stade 1 puis homogénéise,

    3) on incorpore le complément du moyen b) jusqu'à la quantité requise de 40 à 60 parties en poids, sous agitation à une température supérieure ou égale à 110°C, au mélange résultant du stade 2,

    4) on homogénéise le mélange résultant ainsi obtenu à une température supérieure ou égale à 110°C puis le laisse reposer pendant au moins 8 heures,

    5) on chauffe le mélange résultant ainsi obtenu à une température de 50-70°C, de préférence à 60°C, pendant au moins 0,25 h, puis incorpore à cette température le moyen d) et le principe actif dans un solvant dudit principe actif, par exemple l'éthanol,

    6) on homogénéise le mélange résultant pendant au moins 0,5 h sans chauffage,

    7) on dépose le mélange résultant ainsi homogénéisé sur un support temporaire, notamment du papier siliconé à une température de l'ordre de 50-70°C à raison de 100 à 300 g/m², 

    8) on chauffe l'ensemble comprenant ledit support temporaire et la matrice à une température de l'ordre de 70-90°C pour évaporer le solvant du principe actif jusqu'à obtenir un taux résiduel inférieur à 5% en poids,

    9) on transfère la matrice sèche résultante sur un support approprié.

La production industrielle du dispositif selon l'invention est facilitée par le fait que la matrice chargée en principe actif est malléable et également enductible sur un support par la technique dite de "voie fondue", c'est-à-dire par fusion en l'absence de solvant. Quelle que soit la technique d'enduction employée (en phase solvant ou "voie fondue"), on peut ainsi enduire de grandes surfaces puis découper le dispositif à la dimension voulue, calculée en fonction de la quantité de principe actif présent par unité de surface et de la quantité de principe actif à délivrer au sujet pendant un temps déterminé.

Cette technique simple de fabrication par découpage à une surface variable est particulièrement intéressante pour commercialiser des dispositifs de différentes dimensions, donc permettant des apports différents en principe actif. On sait en effet que pour certains principes actifs, notamment les hormones stéroidiennes, les variations individuelles chez les sujets traités sont très grandes et la posologie doit être adaptée à chaque sujet.

L'invention sera mieux comprise à la lecture des exemples de préparation, des tests de perméation et des résultats cliniques qui suivent.

## PREPARATION I

### Exemple 1

On introduit dans un malaxeur de 5 litres 1575 g de LEVAPREN 450P® (EVA ayant une teneur en motifs acétate de vinyle de 45% commercialisé par la Société BAYER) et 980 g d'EUTANOL G® (2-octyldodécanol commercialisé par la Société HENKEL). On chauffe à 140°C et on additionne au mélange, par petites fractions, 350 g d'ETHOCEL 20® (éthylcellulose de viscosité égale à $2 \times 10^{-2}$ Pa · s). Après homogénéisation du milieu, on ajoute 490 g d'EUTANOL G®. On homogénéise l'ensemble pendant 0,5 heure et on laisse reposer pendant 24 heures. On porte ensuite la masse à 60°C pendant 0,5 heure et on additionne une solution de 105 g de dipropylène glycol (DPG) et 175 g de 17-β-oestradiol dans 875 g d'éthanol anhydre. On homogénéise l'ensemble pendant 1 heure sans chauffage. On enduit cette masse sur un papier siliconé de 105 mm de large à une température de 60°C à raison de $190 \pm 5$ g/m². Après passage du papier siliconé enduit à 80°C pour évaporer l'éthanol jusqu'à un taux inférieur à 3,5% en poids, on transfère la matrice sur un support polyéthylène. On découpe alors des formes rectangulaires aux coins arrondis de 20 cm² que l'on conditionne en sachets aluminium/polyéthylène thermosoudés.

## PREPARATION II

### Exemple 2

De façon analogue à la préparation I, on mélange 1575 g de LEVAPREN 450P®, 1050 g d'EUTANOL G® et 175 g d'ETHOCEL 20® à 140°C, puis on ajoute 525 g d'EUTANOL G®. On additionne ensuite, à 60°C, 175 g de dipropylène glycol et 175 g de 17-β-oestradiol dans 875 g d'éthanol anhydre. On procède ensuite de manière identique à la préparation I.

## PREPARATION III

### Exemple 3

De façon analogue à la préparation I, on mélange 1575 g de LEVAPREN 450P®, 990 g d'EUTANOL G® et 175 g d'ETHOCEL 20® à 140°C puis on ajoute 497,5 g d'EUTANOL G®. On additionne ensuite, à 60°C, 262,5 g de dipropylène glycol et 175 g de 17-β-oestradiol dans 875 g d'éthanol anhydre. On procède ensuite de manière identique à la préparation I en enduisant un papier siliconé à raison de $200 \pm 5$ g/m², de façon à laisser un taux d'éthanol résiduel inférieur à 1,5% en poids.

## PREPARATION IV

### Exemple 4

De façon analogue à la préparation I, on mélange 1610 g de LEVAPREN 450P®, 1070 g d'EUTANOL G® et 262,5 g d'ETHOCEL 20® à 140°C, puis on ajoute 540 g d'EUTANOL G®. On additionne ensuite, à 60°C, 17,5 g de dipropylène glycol et 175 g de 17-β-oestradiol dans 875 g d'éthanol anhydre. On procède ensuite de manière identique à la préparation I en enduisant un papier siliconé à raison de $200 \pm 5$ g/m², de façon à laisser un taux d'éthanol résiduel inférieur à 1,5% en poids.

## PREPARATION V

### Exemple 5 : Préparation d'une enduction selon la technique dite de "voie fondue"

Selon un protocole analogue à celui décrit dans la préparation I, on mélange dans un malaxeur de 17 l, 4639 g de LEVAPREN 450P® avec 4330 g d'EUTANOL G® et 1031 g d'ETHOCEL 20®. On chauffe ensuite à 130°C pendant 90 minutes sous agitation moyenne. La masse obtenue est refroidie, laissée au repos à température ambiante pendant 24 h, puis chauffée à 60°C dans un malaxeur. Après ramollissement de la masse, on introduit une suspension constituée par 546 g de 17-β-oestradiol dans 283 g de dipropylène glycol. Le malaxage est poursuivi à 60°C pendant encore 1 heure. La masse polymérique ainsi obtenue est introduite dans un appareil d'enduction type "Hot-melt" et chauffée à 100°C. Cette masse est ensuite enduite sur un support polyester siliconé d'épaisseur 35 μm, de largeur 100 mm, à une température de 105°C et à un grammage de $150 \pm 5$ g/m². Le transfert sur un support polyéthylène s'effectue immediatement après l'enduction. Le complexe obtenu est stabilisé par passage dans un four à 60° ± 5°C.

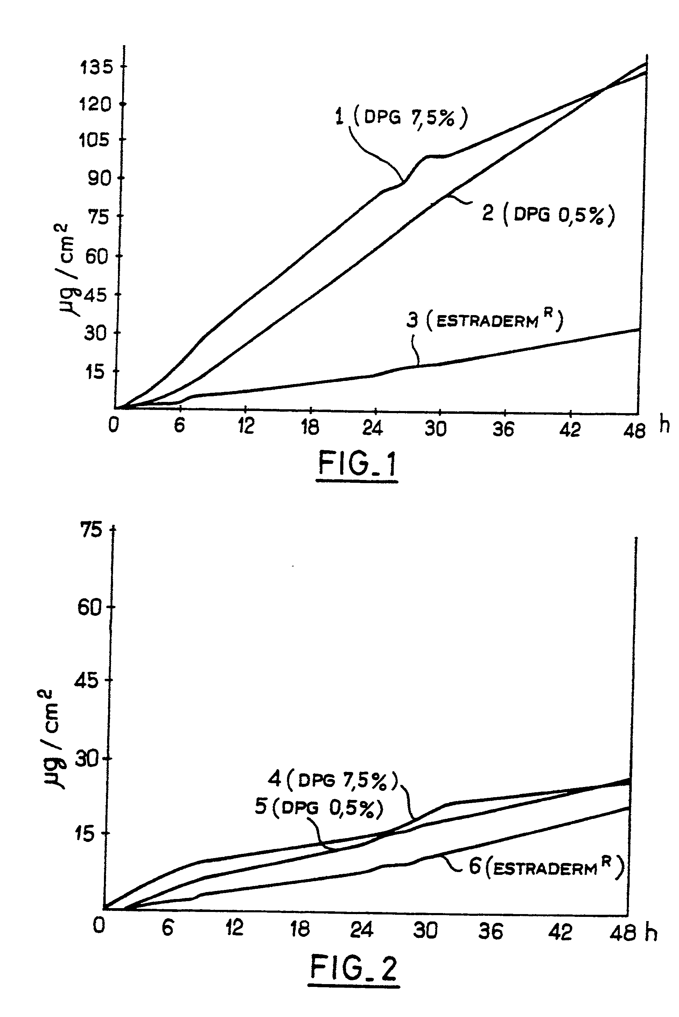

On a réalisé des cinétiques de perméation sur peau de souris "hairless" en cellule statique type "Franz" en éluant avec un mélange sérum physiologique/ethanol/PEG 400 (60/20/20, v/v/v) à 37°C en comparaison avec le produit vendu par la Société CIBA GEIGY sous la marque ESTRADERM® (courbe 3). Les résultats moyens obtenus à partir de 15 échantillons préparés selon les préparations III et IV sont regroupés à la figure 1 où on a représenté le pourcentage d'oestradiol libéré en $\mu g/cm^2$ en fonction du temps en heures (courbe 1 avec DPG 7,5% et courbe 2 avec DPG 0,5%).

Des cinétiques de perméation sur stratum corneum humain ont également été réalisées de manière identique en comparaison avec ESTRADERM® (courbe 6). On a représenté en figure 2 les résultats moyens obtenus à partir de 15 échantillons préparés selon les préparations III et IV (courbe 4 avec DPG 7,5% et courbe 5 avec DPG 0,5%).

Il ressort de ces résultats que le flux d'oestradiol obtenu avec les dispositifs selon l'invention est significativement plus important sur peau de souris et comparable sur stratum corneum humain par rapport au flux obtenu avec ESTRADERM®.

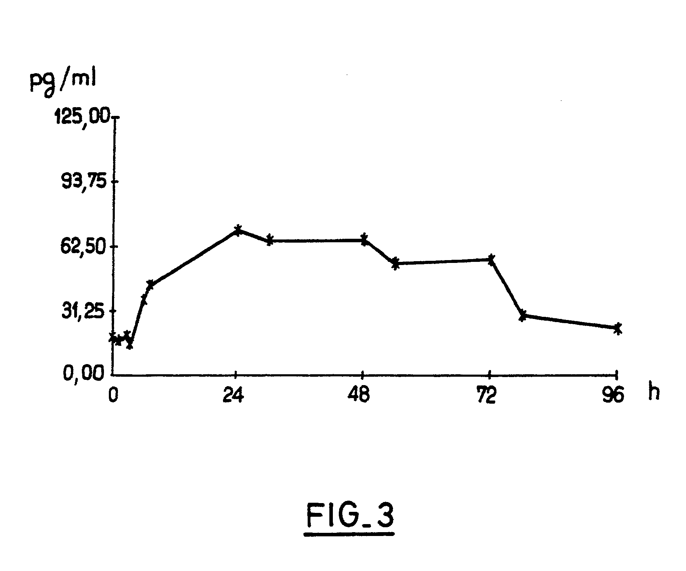

On a représenté sur la figure 3 le taux moyen d'oestradiol plasmatique obtenu chez 6 femmes ménopausées ayant porté pendant trois jours en dispositif de 20 cm² préparé selon la préparation I. On observe que la cinétique moyenne obtenue est comparable à celle obtenue avec le dispositif commercialisé par la Société CIBA GEIGY sous la marque ESTRADERM®, décrite à la figure 5 page 95 dans Journal of Controlled Release, 2 (1985) 89-97. Il apparaît toutefois que le dispositif selon l'invention n'entraîne pas de pic plasmatique en début de traitement. Lors de cette étude, il n'a été noté aucune manifestation d'allergies.

## Revendications

### Revendications pour les Etats contractants suivants : AT, BE, CH, DE, FR, GB, IT, LI, LU, NL, SE

1. Matrice auto-adhésive pour l'administration d'un principe actif par voie percutanée, caractérisée en ce qu'elle comprend :
    a) 40 à 60 parties en poids de copolymère d'éthylène et d'acétate de vinyle,
    b) 40 à 60 parties en poids d'alcool aliphatique supérieur,
    c) 1 à 20 parties en poids de dérivé de cellulose,
    d) 0,1 à 8 parties en poids d'alcool polyhydrique, et
    e) 0,01 à 10 parties en poids de principe actif administrable par voie percutanée,
    le rapport en poids a + c/b + d étant compris entre 0,7 et 1,3.

2. Matrice auto-adhésive pour l'administration d'un principe actif par voie percutanée, caractérisée en ce qu'elle comprend :
    a) 40 à 60 parties en poids de copolymère d'éthylène et d'acétate de vinyle,
    b) 40 à 60 parties en poids d'alcool aliphatique supérieur,
    c) 1 à 20 parties en poids de dérivé de cellulose,
    d) 0,1 à 8 parties en poids d'alcool polyhydrique, et,
    e) 0,01 à 10 parties en poids d'un stéroïde choisi parmi l'oestradiol, la progestérone, la testostérone et leurs dérivés, et les corticostéroïdes,
    le rapport en poids a + c/b + d étant compris entre 0,7 et 1,3.

3. Matrice suivant les revendications 1 ou 2, caractérisée en ce que le copolymère d'éthylène et d'acétate de vinyle a une teneur en motifs acétate de vinyle comprise entre 35 et 55% en poids par rapport au poids dudit copolymère.

4. Matrice suivant la revendication 3, caractérisée en ce que le copolymère d'éthylène et d'acétate de vinyle a une teneur en motifs acétate de vinyle de l'ordre de 45% en poids par rapport au poids dudit copolymère.

5. Matrice suivant les revendications 1 ou 2, caractérisée en ce que l'alcool aliphatique supérieur est choisi parmi l'ensemble comprenant les mono-alcools saturés ou insaturés ayant de 12 à 20 atomes de carbone.

6. Matrice suivant les revendications 1 ou 2, caractérisée en ce que le dérivé de cellulose est choisi parmi l'ensemble comprenant les alkylcelluloses et hydroxyalkylcelluloses, notamment les méthylcellulose, éthylcellulose, propylcellulose, méthylpropylcellulose, hydroxyméthylcellulose, hydroxyéthylcellulose et hydroxypropylcellulose.

7. Matrice suivant les revendications 1 ou 2, caractérisée en ce que l'alcool polyhydrique est un glycol.

8. Matrice suivant la revendication 7, caractérisée en ce que le glycol est choisi parmi l'ensemble comprenant les alkylène glycols, notamment les éthylène glycol, propylène glycol, butylène glycol, triéthylène glycol, diéthylène glycol, polyéthylène glycol, polypropylène glycol.

9. Matrice suivant les revendications 1 ou 2, caractérisée en ce qu'elle comprend :
    a) 40 à 60 parties en poids de copolymère d'éthylène et d'acétate de vinyle,
    b) 40 à 60 parties en poids de 2-octyl-1-dodécanol,
    c) 1 à 20 parties en poids d'éthylcellulose,
    d) 0,1 à 8 parties en poids de dipropylène glycol, et,
    e) 0,01 à 10 parties en poids d'oestradiol,
    le rapport en poids a + c/b + d étant compris entre 0,7 et 1,3.

10. Matrice suivant les revendications 1 ou 2, caractérisée en ce qu'elle comprend :

a) 45 parties en poids d'un copolymère éthylène-acétate de vinyle comportant 45% en poids de motifs acétate de vinyle par rapport au poids dudit copolymère,

b) 40 à 45 parties en poids de 2-octyl-1-dodécanol,

c) 5 à 10 parties en poids d'éthylcellulose de viscosité comprise entre $2 \times 10^{-2}$ et $2 \times 10^{-1}$ Pa · s,

d) 1 à 5 parties en poids de dipropylène glycol, et,

e) 3 à 5 parties en poids de β-oestradiol.

11. Procédé de préparation d'une matrice suivant les revendications 1 ou 2, caractérisé en ce que :

1) on mélange sous agitation le moyen a) et une portion du moyen b) inférieure à la quantité requise de 40 à 60 parties en poids à une température supérieure ou égale à 110°C,

2) on incorpore, sous agitation, à une température supérieure ou égale à 110°C, le moyen c) dans le mélange résultant du stade 1 puis homogénéise,

3) on incorpore le complément du moyen b) jusqu'à la quantité requise de 40 à 60 parties en poids, sous agitation à une température supérieure ou égale à 110°C, au mélange résultant du stade 2,

4) on homogénéise le mélange résultant ainsi obtenu à une température supérieure ou égale à 110°C puis le laisse reposer pendant au moins 8 heures,

5) on chauffe le mélange résultant ainsi obtenu à une température de 50-70°C, de préférence à 60°C, pendant au moins 0,25 h, puis incorpore à cette température le moyen d) et le principe actif dans un solvant dudit principe actif,

6) on homogénéise le mélange résultant pendant au moins 0,5 h sans chauffage,

7) on dépose le mélange résultant ainsi homogénéisé sur un support temporaire, notamment du papier siliconé à une température de 50-70°C à raison de 100 à 300 g/m2,

8) on chauffe l'ensemble comprenant ledit support temporaire et la matrice à une température de 70-90°C pour évaporer le solvant du principe actif jusqu'à obtenir un taux résiduel inférieur à 5% en poids,

9) on transfère la matrice sèche résultante sur un support approprié.

## Revendications pour les Etats contractants suivants : ES, GR

1. Procédé de préparation d'une matrice autoadhésive pour l'administration d'un principe actif par voie percutanée, et comprenant :

a) 40 à 60 parties en poids de copolymère d'éthylène et d'acétate de vinyle,

b) 40 à 60 parties en poids d'alcool aliphatique supérieur,

c) 1 à 20 parties en poids de dérivé de cellulose,

d) 0,1 à 8 parties en poids d'alcool polyhydrique, et

e) 0,01 à 10 parties en poids de principe actif administrable par voie percutanée,

le rapport en poids a + c/b + d étant compris entre 0,7 et 1,3 ; ledit procédé étant caractérisé en ce que :

1) on mélange sous agitation le moyen a) et une portion du moyen b) inférieure à la quantité requise de 40 à 60 parties en poids, à une température supérieure ou égale à 110°C,

2) on incorpore, sous agitation, à une température supérieure ou égale à 110°C, le moyen c) dans le mélange résultant du stade 1 puis homogénéise,

3) on incorpore le complément du moyen b) jusqu'à la quantité requise de 40 à 60 parties en poids, sous agitation à une température supérieure ou égale à 110°C, au mélange résultant du stade 2,

4) on homogénéise le mélange résultant ainsi obtenu à une température supérieure ou égale à 110°C puis le laisse reposer pendant au moins 8 heures,

5) on chauffe le mélange résultant ainsi obtenu à une température de 50-70°C, de préférence à 60°C, pendant au moins 0,25 h, puis incorpore à cette température le moyen d) et le principe actif dans un solvant dudit principe actif,

6) on homogénéise le mélange résultant pendant au moins 0,5 h sans chauffage,

7) on dépose le mélange résultant ainsi homogénéisé sur un support temporaire, notamment du papier siliconé à une température de 50-70°C à raison de 100 à 300 g/m2,

8) on chauffe l'ensemble comprenant ledit support temporaire et la matrice à une température de 70-90°C pour évaporer le solvant du principe actif jusqu'à obtenir un taux résiduel inférieur à 5% en poids,

9) on transfère la matrice sèche résultante sur un support approprié.

2. Procédé suivant la revendication 1, caractérisé en ce que le principe actif est un stéroïde choisi parmi l'oestradiol, la progestérone, la testostérone et leurs dérivés, et les corticostéroïdes.

3. Procédé suivant la revendication 1, caractérisé en ce que le copolymère d'éthylène et d'acétate de vinyle a une teneur en motifs acétate de vinyle comprise entre 35 et 55% en poids par rapport au poids dudit copolymère.

4. Procédé suivant la revendication 1, caractérisé en ce que le copolymère d'éthylène et d'acétate de vinyle a une teneur en motifs acétate de vinyle de l'ordre de 45% en poids par rapport au poids dudit copolymère.

5. Procédé suivant la revendication 1, caractérisé en ce que l'alcool aliphatique supérieur est choisi parmi l'ensemble comprenant les mono-alcools saturés ou insaturés ayant de 12 à 20 atomes de carbone.

6. Procédé suivant la revendication 1, caractérisé en ce que le dérivé de cellulose est choisi parmi l'ensemble comprenant les alkylcelluloses et hydroxyalkylcelluloses, notamment les méthylcellulose, éthylcellulose, propylcellulose, méthylpropylcellulose, hydroxyméthylcellulose, hydroxyéthylcellulose et hydroxypropylcellulose.

7. Procédé suivant la revendication 1, caractérisé en ce que l'alcool polyhydrique est un glycol.

8. Procédé suivant la revendication 7, caractérisé en ce que le glycol est choisi parmi l'ensemble comprenant les alkylène glycols, notamment les éthylène glycol, propylène glycol, butylène glycol, triéthylène glycol, diéthylène glycol, polyéthylène glycol, polypropylène glycol.

9. Procédé suivant la revendication 1, dans lequel on utilise

    a) 45 parties en poids d'un copolymère éthylène-acétate de vinyle comportant 45% en poids de motifs acétate de vinyle par rapport au poids dudit copolymère,

    b) 40 à 45 parties en poids de 2-octyl-1-dodécanol,

    c) 5 à 10 parties en poids d'éthylcellulose de viscosité comprise entre $2 \times 10^{-2}$ et $2 \times 10^{-1}$ Pa · s,

    d) 1 à 5 parties en poids de dipropylène glycol, et,

    e) 3 à 5 parties en poids de β-oestradiol.

## Patentansprüche

### Patentansprüche für folgende Vertragsstaaten : AT, BE, CH, DE, FR, GB, IT, LI, LU, NL, SE

1. Selbstklebende Matrix für die transdermale Verabreichung eines Wirkstoffs, dadurch gekennzeichnet, daß sie umfaßt :

    a) 40 bis 60 Gew.-Teile Ethylen/Vinylacetat-Copolymer,

    b) 40 bis 60 Gew.-Teile höheren aliphatischen Alkohol,

    c) 1 bis 20 Gew.-Teile Cellulosederivat,

    d) 0,1 bis 8 Gew.-Teile mehrwertigen Alkohol und

    e) 0,01 bis 10 Gew.-Teile transdermal verabreichbaren Wirkstoff,

    wobei das Gewichtsverhältnis a + c/b + d zwischen 0,7 und 1,3 liegt.

2. Selbstklebende Matrix zur transdermalen Verabreichung eines Wirkstoffs, dadurch gekennzeichnet, daß sie umfaßt :

    a) 40 bis 60 Gew.-Teile Ethylen/Vinylacetat-Copolymer,

    b) 40 bis 60 Gew.-Teile höheren aliphatischen Alkohol,

    c) 1 bis 20 Gew.-Teile Cellulosederivat,

    d) 0,1 bis 8 Gew.-Teile mehrwertigen Alkohol und

    e) 0,01 bis 10 Gew.-Teile eines aus Östradiol, Progesteron, Testosteron und ihren Derivaten sowie den Corticosteroiden ausgewählten Steroids,

    wobei das Gewichtsverhältnis a + c/b + d zwischen 0,7 und 1,3 liegt.

3. Matrix nach Anspruch 1 oder 2, dadurch gekennzeichnet, daß das Ethylen/Vinylacetat-Copolymer einen Gehalt an Vinylacetatgruppen zwischen 35 und 55 Gew.-%, bezogen auf das Gewicht des Polymers, aufweist.

4. Matrix nach Anspruch 3, dadurch gekennzeichnet, daß das Ethylen/Vinylacetat-Copolymer einen Gehalt an Vinylacetatgruppen in der Größenordnung von 45%, bezogen auf das Gewicht des Copolymers, aufweist.

5. Matrix nach Anspruch 1 oder 2, dadurch gekennzeichnet, daß der höhere aliphatische Alkohol aus der aus gesättigten oder ungesättigten Monoalkoholen mit 12 bis 20 Kohlenstoffatomen bestehenden Gruppe ausgewählt ist.

6. Matrix nach Anspruch 1 oder 2, dadurch gekennzeichnet, daß das Cellulosederivat aus der aus Alkylcellulosen und Hydroxyalkylcellulosen, insbesondere Methylcellulose, Ethylcellulose, Propylcellulose, Methylpropylcellulose, Hydroxymethylcellulose, Hydroxyethylcellulose und Hydroxypropylcellulose bestehenden Gruppe ausgewählt ist.

7. Matrix nach Anspruch 1 oder 2, dadurch gekennzeichnet, daß der mehrwertige Alkohol ein Glykol ist.

8. Matrix nach Anspruch 7, dadurch gekennzeichnet, daß das Glykol aus der Alkylglykolen, insbesondere Ethylenglykol, Propylenglykol, Butylenglykol, Triethylenglykol, Diethylenglykol, Polyethylenglykol, Polypropylenglykol, bestehenden Gruppe ausgewählt ist.

9. Matrix nach Anspruch 1 oder 2, dadurch gekennzeichnet, daß sie umfaßt :

    a) 40 bis 60 Gew.-Teile Ethylen/Vinylacetat-Copolymer,

    b) 40 bis 60 Gew.-Teile Oct-2-yl-dodecan-1-ol,

    c) 1 bis 20 Gew.-Teile Ethylcellulose,

    d) 0,1 bis 8 Gew.-Teile Dipropylenglykol und

    e) 0,01 bis 10 Gew.-Teile Östradiol,

    wobei das Gewichtsverhältnis a + c/b + d zwischen 0,7 und 1,3 liegt.

10. Matrix nach Anspruch 1 oder 2, dadurch gekennzeichnet, daß sie umfaßt :

    a) 45 Gew.-Teile eines Ethylen/Vinylacetat-Copolymers mit 45 Gew.-% Vinylacetatgruppen, bezogen auf das Gewicht des Copolymers,

    b) 40 bis 45 Gew.-Teile Oct-2-yl-dodecan-1-ol,

    c) 5 bis 10 Gew.-Teile Ethylcellulose mit einer

Viskosität zwischen $2 \times 10^{-2}$ und $2 \times 10^{-1}$ Pa · s,

d) 1 bis 5 Gew.-Teile Dipropylenglykol und

e) 3 bis 5 Gew.-Teile β-Östradiol.

11. Verfahren zur Herstellung einer Matrix nach Anspruch 1 oder 2, dadurch gekennzeichnet, daß

1) man den Bestandteil a) und einen Teil des Bestandteils b), der kleiner ist als die benötigte Menge von 40 bis 60 Gew.-Teilen, bei einer Temperatur von mehr als oder gleich 110°C unter Rühren mischt,

2) man unter Rühren bei einer Temperatur von mehr als oder gleich 110°C den Bestandteil c) in die aus der Stufe 1) resultierende Mischung einbringt und danach homogenisiert,

3) man den Rest des Bestandteils b) bis zur benötigten Menge von 40 bis 60 Gew.-Teilen unter Rühren bei einer Temperatur von mehr als oder gleich 110°C in die aus Stufe 2) resultierende Mischung einbringt,

4) man die so erhaltene resultierende Mischung bei einer Temperatur von mehr als oder gleich 110°C homogenisiert und danach wenigstens 8 Stunden ruhen läßt,

5) man die so erhaltene resultierende Mischung bei einer Temperatur von 50 bis 70°C, vorzugsweise von 60°C, über wenigstens 0,25 h erhitzt, danach bei dieser Temperatur den Bestandteil d) und den Wirkstoff in einem Lösungsmittel für den Wirkstoff einbringt,

6) man die resultierende Mischung über wenigstens 0,5 h ohne Erhitzen homogenisiert,

7) man die so homogenisierte resultierende Mischung auf einem zeitweiligen Träger deponiert, insbesondere auf Silikonpapier bei einer Temperatur von 50 bis 70°C in einem Verhältnis von 100 bis 300 g/m²,

8) man das Ganze mit dem zeitweiligen Träger und der Matrix auf eine Temperatur von 70 bis 90°C erhitzt, um das Lösungsmittel des Wirkstoffs bis zu einem Restgehalt von weniger als 5 Gew.-% zu verdampfen und

9) man die resultierende trockene Matrix auf einen geeigneten Träger transferiert.

**Patentansprüche für folgende Vertragsstaaten : ES, GR**

1. Verfahren zur Herstellung einer selbstklebenden Matrix für die transdermale Verabreichung eines Wirkstoffs, welche umfaßt :

a) 40 bis 60 Gew.-Teile Ethylen/Vinylacetat-Copolymer,

b) 40 bis 60 Gew.-Teile höheren aliphatischen Alkohol,

c) 1 bis 20 Gew.-Teile Cellulosederivat,

d) 0,1 bis 8 Gew.-Teile mehrwertigen Alkohol, und

e) 0,01 bis 10 Gew.-Teile transdermal verabreichbaren Wirkstoff,

wobei das Gewichtsverhältnis a + c/b + d zwischen 0,7 und 1,3 liegt, welches Verfahren dadurch gekennzeichnet ist, daß

1) man den Bestandteil a) und einen Teil des Bestandteils b), der kleiner ist als die benötigte Menge von 40 bis 60 Gew.-Teilen, bei einer Temperatur von mehr als oder gleich 110°C unter Rühren mischt,

2) man unter Rühren bei einer Temperatur von mehr als oder gleich 110°C den Bestandteil c) in die aus der Stufe 1) resultierende Mischung einbringt und danach homogenisiert,

3) man den Rest des Bestandteils b) bis zur benötigten Menge von 40 bis 60 Gew.-Teilen unter Rühren bei einer Temperatur von mehr als oder gleich 110°C in die aus Stufe 2) resultierende Mischung einbringt,

4) man die so erhaltene resultierende Mischung bei einer Temperatur von mehr als oder gleich 110°C homogenisiert und danach wenigstens 8 Stunden ruhen läßt,

5) man die so erhaltene resultierende Mischung bei einer Temperatur von 50 bis 70°C, vorzugsweise von 60°C, über wenigstens 0,25 h erhitzt, danach bei dieser Temperatur den Bestandteil d) und den Wirkstoff in einem Lösungsmittel für den Wirkstoff einbringt,

6) man die resultierende Mischung über wenigstens 0,5 h ohne Erhitzen homogenisiert,

7) man die so homogenisierte resultierende Mischung auf einem zeitweiligen Träger deponiert, insbesondere auf Silikonpapier bei einer Temperatur von 50 bis 70°C in einem Verhältnis von 100 bis 300 g/m²,

8) man das Ganze mit dem zeitweiligen Träger und der Matrix auf eine Temperatur von 70 bis 90°C erhitzt, um das Lösungsmittel des Wirkstoffs bis zu einem Restgehalt von weniger als 5 Gew.-% zu verdampfen und

9) man die resultierende trockene Matrix auf einen geeigneten Träger transferiert.

2. Verfahren nach Anspruch 1, dadurch gekennzeichnet, daß der Wirkstoff ein aus Östradiol, Progesteron, Testosteron und ihren Derivaten sowie den Corticosteroiden ausgewähltes Steroid ist.

3. Verfahren nach Anspruch 1, dadurch gekennzeichnet, daß das Ethylen/Vinylacetat-Copolymer einen Gehalt an Vinylacetatgruppen zwischen 35 und 55 Gew.-%, bezogen auf das Gewicht des Copolymers, aufweist.

4. Verfahren nach Anspruch 1, dadurch gekennzeichnet, daß das Ethylen/Vinylacetat-Copolymer einen Gehalt an Vinylacetatgruppen in der Größenordnung von 45 Gew.-%, bezogen auf das Gewicht des Copolymers, aufweist.

5. Verfahren nach Anspruch 1, dadurch gekennzeichnet, daß der höhere aliphatische Alkohol aus der aus gesättigten oder ungesättigten Monoalkoholen mit 12 bis 20 Kohlenstoffatomen bestehenden Gruppe ausgewählt ist.

6. Verfahren nach Anspruch 1, dadurch gekennzeichnet, daß das Cellulosederivat aus der aus Alkylcellulosen und Hydroxyalkylcellulosen, insbesondere Methylcellulose, Ethylcellulose, Propylcellulose, Methylpropylcellulose, Hydroxymethylcellulose, Hydroxyethylcellulose und Hydroxypropylcellulose, bestehenden Gruppe ausgewählt ist.

7. Verfahren nach Anspruch 1, dadurch gekennzeichnet, daß der mehrwertige Alkohol ein Glykol ist.

8. Verfahren nach Anspruch 7, dadurch gekennzeichnet, daß das Glykol aus der aus Alkylenglykolen, insbesondere Ethylenglykol, Propylenglykol, Butylenglykol, Triethylenglykol, Diethylenglykol, Polyethylenglykol, Polypropylenglykol, bestehenden Gruppe ausgewählt ist.

9. Verfahren nach Anspruch 1, in welchem man
a) 45 Gew.-Teile eines Ethylen/Vinylacetat-Copolymers mit 45 Gew.-% Vinylacetatgruppen, bezogen auf das Gewicht des Copolymers,
b) 40 bis 45 Gew.-Teile Oct-2-yl-dodecan-1-ol,
c) 5 bis 10 Gew.-Teile Ethylcellulose einer Viskosität zwischen $2 \times 10^{-2}$ und $2 \times 10^{-1}$ Pa · s,
d) 1 bis 5 Gew.-Teile Dipropylenglykol und
e) 3 bis 5 Gew.-Teile β-Östradiol verwendet.

## Claims

### Claims for the following Contracting states : AT, BE, CH, DE, FR, GB, IT, LI, LU, NL, SE

1. A self-adhesive matrix for the percutaneous administration of an active ingredient, which comprises :
a) 40 to 60 parts by weight of an ethylene/vinyl acetate copolymer material,
b) 40 to 60 parts by weight of a higher aliphatic alcohol compound,
c) 1 to 20 parts by weight of a cellulose derivative material,
d) 0.1 to 8 parts by weight of a polyhydric alcohol compound, and
e) 0.01 to 10 parts by weight of an active ingredient which can be administered percutaneously, the weight ratio a + c/b + d being between 0.7 and 1.3.

2. A self-adhesive matrix for the percutaneous administration of an active ingredient, which comprises :
a) 40 to 60 parts by weight of an ethylene/vinyl acetate copolymer material,
b) 40 to 60 parts by weight of a higher aliphatic alcohol compound,
c) 1 to 20 parts by weight of a cellulose derivative material,

d) 0.1 to 8 parts by weight of a polyhydric alcohol compound, and
e) 0.01 to 10 parts by weight of a steroid selected from the group consisting of estradiol, progesterone, testosterone and derivative materials thereof, and corticosteroids, the weight ratio a + c/b + d being between 0.7 and 1.3.

3. The matrix as claimed in claim 1 or 2, wherein the ethylene/vinyl acetate copolymer material has a content of vinyl acetate units of between 35 and 55% by weight, relative to the weight of the said copolymer material.

4. The matrix as claimed in claim 3, wherein the ethylene/vinyl acetate copolymer material has a content of vinyl acetate units of the order of 45% by weight, relative to the weight of the said copolymer material.

5. The matrix as claimed in claim 1 or 2, wherein the higher aliphatic alcohol compound is selected from the group consisting of saturated or unsaturated monoalcohol compounds having from 12 to 20 carbon atoms.

6. The matrix as claimed in claim 1 or 2, wherein the cellulose derivative material is selected from the group consisting of alkyl celluloses and hydroxyalkyl celluloses, in particular methyl cellulose, ethyl cellulose, propyl cellulose, methylpropyl cellulose, hydroxymethyl cellulose, hydroxyethyl cellulose and hydroxypropyl cellulose.

7. The matrix as claimed in claim 1 or 2, wherein the polyhydric alcohol compound is a glycol compound.

8. The matrix as claimed in claim 7, wherein the glycol compound is selected from the group consisting of ethylene glycol, propylene glycol, butylene glycol, triethylene glycol, diethylene glycol, polyethylene glycol and polypropylene glycol.

9. The matrix as claimed in claim 1 or 2, which comprises :
a) 40 to 60 parts by weight of an ethylene/vinyl acetate copolymer material,
b) 40 to 60 parts by weight of 2-octyldodecan-1-ol,
c) 1 to 20 parts by weight of ethyl cellulose,
d) 0.1 to 8 parts by weight of dipropylene glycol, and
e) 0.01 to 10 parts by weight of estradiol, the weight ratio a + c/b + d being between 0.7 and 1.3.

10. The matrix as claimed in claim 1 or 2, which comprises :
a) 45 parts by weight of an ethylene/vinyl acetate copolymer containing 45% by weight of vinyl acetate units, relative to the weight of the said copolymer material,
b) 40 to 45 parts by weight of 2-octyldodecan-1-ol,

c) 5 to 10 parts by weight of ethyl cellulose with a viscosity of between $2 \times 10^{-2}$ and $2 \times 10^{-1}$ Pa · s,

d) 1 to 5 parts by weight of dipropylene glycol, and

e) 3 to 5 parts by weight of β-estradiol.

11. A method for the preparation of a matrix as claimed in claim 1 or 2, wherein :

1) the means a) and a portion of the means b), lower than the required amount of 40-60 parts by weight, are mixed, with stirring, at a temperature greater than or equal to 110°C,

2) the means (c) is incorporated into the mixture resulting from stage 1, with stirring, at a temperature greater than or equal to 110°C, and then homogenized,

3) the remainder of the means b) up to be required amount of 40-60 parts by weight is incorporated into the mixture resulting from stage 2, with stirring, at a temperature greater than or equal to 110°C,

4) the resulting mixture obtained in this way is homogenized at a temperature greater than or equal to 110°C and then left to stand for at least 8 hours,

5) the resulting mixture obtained in this way is heated at a temperature of 50-70°C, preferably 60°C, for at least 0.25 h, after which the means d) and the active ingredient in a solvent for the said active ingredient are then incorporated at this temperature,

6) the resulting mixture is homogenized for at least 0.5 h without heating,

7) the resulting mixture homogenised in this way is deposited on a temporary support, especially silicone-treated paper, at a temperature of the order of 50-70°C, at a rate of 100 to 300 $g/m^2$,

8) the whole comprising the said temporary support and the matrix is heated at a temperature of 70-90°C in order to evaporate the solvent for the active ingredient until the residual proportion is less than 5% by weight, and

9) the resulting dry matrix is transferred onto an appropriate support.

**Claims for the following contracting states :
ES, GR**

1. A method for the preparation of a self-adhesive matrix for the percutaneous administration of an active ingredient, which comprises :

a) 40 to 60 parts by weight of an ethylene/vinyl acetate copolymer material,

b) 40 to 60 parts by weight of a higher aliphatic alcohol compound,

c) 1 to 20 parts by weight of a cellulose derivative material,

d) 0.1 to 8 parts by weight of a polyhydric alcohol compound, and

e) 0.01 to 10 parts by weight of an active ingredient which can be administered percutaneously, the weight ratio a + c/b + d being between 0.7 and 1.3, wherein :

1) the means a) and a portion of the means b), lower than the required amount of 40-60 parts by weight, are mixed, with stirring, at a temperature greater than or equal to 110°C,

2) the means (c) is incorporated into the mixture resulting from stage 1 with stirring, at a temperature greater than or equal to 110°C, and then homogenized,

3) the remainder of the means b) up to be required amount of 40-60 parts by weight is incorporated into the mixture resulting from stage 2, with stirring, at a temperature greater than or equal to 110°C,

4) the resulting mixture obtained in this way is homogenized at a temperature greater than or equal to 110°C and then left to stand for at least 8 hours,

5) the resulting mixture obtained in this way is heated at a temperature of 50-70°C, preferably 60°C, for at least 0.25 h, after which the means d) and the active ingredient in a solvent for the said active ingredient are then incorporated at this temperature,

6) the resulting mixture is homogenized for at least 0.5 h without heating,

7) the resulting mixture homogenized in this way is deposited on a temporary support, especially silicone-treated paper, at a temperature of the order of 50-70°C, at a rate of 100 to 300 $g/m^2$,

8) the whole comprising the said temporary support and the matrix is heated at a temperature of 70-90°C in order to evaporate the solvent for the active ingredient until the residual proportion is less than 5% by weight, and 9) the resulting dry matrix is transferred onto an appropriate support.

2. A method according to claim 1, wherein the active ingredient is a steroid selected from the group consisting of estradiol, progesterone, testosterone and derivative materials thereof, and corticosteroids.

3. A method according to claim 1, wherein the ethylene/vinyl acetate copolymer material has a content of vinyl acetate units of between 35 and 55% by weight, relative to the weight of the said copolymer material.

4. A method according to claim 1, wherein the ethylene/vinyl acetate copolymer material has a content of vinyl acetate units of the order of 45% by weight, relative to the weight of the said copolymer material.

5. A method according to claim 1, wherein the higher aliphatic alcohol compound is selected from the group consisting of saturated or unsaturated monoalcohol compounds having from 12 to 20 carbon atoms.

6. A method according to claim 1, wherein the cellulose derivative material is selected from the group consisting of alkyl celluloses and hydroxyalkyl celluloses, in particular methyl cellulose, ethyl cellulose, propyl cellulose, methylpropyl cellulose, hydroxymethyl cellulose, hydroxyethyl cellulose and hydroxypropyl cellulose.

7. A method according to claim 1, wherein the polyhydric alcohol compound is a glycol compound.

8. A method according to claim 7, wherein the glycol compound is selected from the group consisting of ethylene glycol, propylene glycol, butylene glycol, triethylene glycol, diethylene glycol, polyethylene glycol and polypropylene glycol.

9. A method according to claim 1, wherein are used

   a) 45 parts by weight of an ethylene/vinyl acetate copolymer containing 45% by weight of vinyl acetate units, relative to the weight of the said copolymer material,

   b) 40 to 45 parts by weight of 2-octyldodecan-1-ol,

   c) 5 to 10 parts by weight of ethyl cellulose with a viscosity of between $2 \times 10^{-2}$ and $2 \times 10^{-1}$ Pa · s,

   d) 1 to 5 parts by weight of dipropylene glycol, and

   e) 3 to 5 parts by weight of β-estradiol.

FIG_1

FIG_2

FIG_3